(19) Europäisches Patentamt
European Patent Office
Office européen des brevets

(11) Numéro de publication: **0 130 637**
**A2**

(12) **DEMANDE DE BREVET EUROPEEN**

(21) Numéro de dépôt: **84200864.1**

(22) Date de dépôt: **14.06.84**

(51) Int. Cl.⁴: **C 07 C 69/734,** C 07 C 121/30, C 07 C 149/237

(30) Priorité: **24.06.83 BE 897134**

(43) Date de publication de la demande: **09.01.85 Bulletin 85/2**

(84) Etats contractants désignés: **AT BE CH DE FR GB IT LI LU NL SE**

(71) Demandeur: **SOLVAY & Cie (Société Anonyme), Rue du Prince Albert, 33, B-1050 Bruxelles (BE)**

(72) Inventeur: **Viehe, Heinz, Clos du Paradis, 7, Grand Manil B-1350 Limal (BE)**
Inventeur: **Stevenart-de Mesmaeker, Nadine, Rue E. Goes, B-1348 Louvain-la-Neuve (BE)**
Inventeur: **Merenyi, Robert, Rankiaan, 3, B-1900 Overijse-Maleizen (BE)**

(54) **Diènes polysubstitués.**

(57) Les diènes polysubstitués selon l'invention répondent à la formule générale

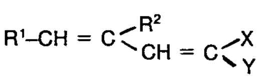

dans laquelle:

– X représente un groupement sélectionné parmi les groupements SH, $SR^3$, $OR^3$, $SeR^3$, NHR et N = $R^3$ dans lesquels $R^3$ représente un groupement aliphatique ou cyclique, saturé ou non, comptant de 1 à 20 atomes de carbone,

– Y représente un groupement sélectionné parmi les groupements C ≡ N et 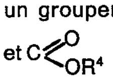 où $R^4$ représente l'hydrogène ou un groupement aliphatique ou cyclique, saturé ou non, comptant de 1 à 20 atomes de carbone, sous réserve que lorsque X représente le groupement $SCH_3$, Y ne représente pas le groupement 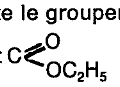 et que lorsque X représente un groupement $OR^3$ où $R^3$ est un groupement alkyle comprenant au moins trois atomes de carbone, Y ne représente pas le groupement C ≡ N,

– $R^1$ représente l'hydrogène, un halogène ou un groupement aliphatique, substitué ou non, comptant de 2 à 8 atomes de carbone,

– $R^2$ représente l'hydrogène, un halogène ou un groupement aliphatique, substitué ou non, et comptant de 1 à 8 atomes de carbone ou un groupement aromatique, substitué ou non, et comptant de 5 à 8 atomes de carbone.

EP 0 130 637 A2

Diènes polysubstitués

Cas S.83/18

SOLVAY & Cie (Société Anonyme)

La présente invention concerne des diènes polysubstitués répondant à la formule générale

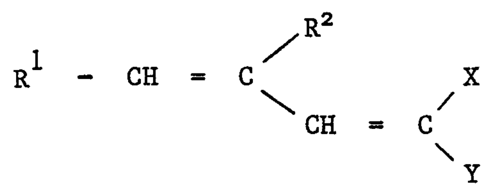

dans laquelle :

- X représente un groupement sélectionné parmi les groupements SH, $SR^3$, $OR^3$, $SeR^3$, $NHR^3$ et $N = R^3$ dans lesquels $R^3$ représente un groupement aliphatique ou cyclique, saturé ou non, comptant de 1 à 20 atomes de carbone,
- Y représente un groupement sélectionné parmi les groupements $C \equiv N$ et 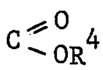 où $R^4$ représente l'hydrogène ou un groupement aliphatique ou cyclique, saturé ou non, comptant de 1 à 20 atomes de carbone, sous réserve que lorsque X représente le groupement $SCH_3$, Y ne représente pas le groupement 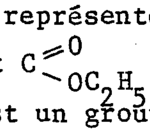 et que lorsque X représente un groupement $OR^3$ où R3 est un groupement alkyle comprenant au moins trois atomes de carbone, Y ne représente pas le groupement $C \equiv N$,
- $R^1$ représente l'hydrogène, un halogène ou un groupement aliphatique, substitué ou non, comptant de 2 à 8 atomes de carbone,
- $R^2$ représente l'hydrogène, un halogène, un groupement aliphatique, substitué ou non, et comptant de 1 à 8 atomes de carbone ou un groupement aromatique, substitué ou non, et comptant de 5 à 8 atomes de carbone.

Habituellement, X représente un groupement sélectionné parmi les groupements $SR^3$, $OR^3$ et $NHR^3$. Le plus souvent $R^3$ représente un groupement alkyle, substitué ou non, comptant de 1 à 6 atomes de

carbone; de préférence X représente un groupement $SCH_3$ ou $OCH_3$. De manière particulièrement préférée, X représente le groupement méthoxy $OCH_3$.

Habituellement, Y représente un groupement sélectionné parmi les groupements $C \equiv N$ et $C\leqslant^{O}_{OR^4}$ où $R^4$ représente l'hydrogène ou un groupement alkyle, cycloalkyle, alcényle ou cycloalcényle, substitué ou non, et comptant jusqu'à 12 atomes de carbone ou un groupement phényle, substitué ou non, comptant de 6 à 10 atomes de carbone étant entendu que les réserves déjà exprimées sont toujours d'application. De manière préférée, Y représente un groupement sélectionné parmi les groupements $C \equiv N$ et $C\leqslant^{O}_{OR^4}$ où $R^4$ représente l'hydrogène ou un groupement alkyle, substitué ou non, comptant de 1 à 3 atomes de carbone ou un groupement phényle, substitué ou non, comptant de 6 à 8 atomes de carbone. De manière particulièrement préférée, Y représente un groupement sélectionné parmi les groupements $C \equiv N$ et $C\leqslant^{O}_{OCH_3}$. De manière tout particulièrement préférée, Y représente le groupement $C\leqslant^{O}_{OCH_3}$.

Habituellement $R^1$ représente l'hydrogène ou un groupement alkyle, alcoxy, alcényle ou alcényloxy, substitué ou non, et comptant de 2 à 6 atomes de carbone. De manière préférée, $R^1$ représente l'hydrogène ou un groupement alkyle ou alcényle comptant de 2 à 4 atomes de carbone; de manière particulièrement préférée, $R^1$ représente l'hydrogène.

Habituellement $R^2$ représente l'hydrogène ou un groupement alkyle, alcoxy, alcényle ou alcényloxy, substitué ou non, et comptant de 1 à 6 atomes de carbone ou un groupement phényle, phénoxy, phénylalkyle ou phénylalcoxy, substitué ou non et comptant de 5 à 8 atomes de carbone. De manière préférée, $R^2$ représente l'hydrogène, un groupement méthoxy, un groupement alkyle comptant de 1 à 3 atomes de carbone ou un groupement phényle comportant 6 atomes de carbone; de manière particulièrement préférée, $R^2$ représente l'hydrogène.

Les diènes polysubstitués selon l'invention peuvent se présenter sous forme d'isomères divers; ils sont utilisables comme intermédiaires de synthèses chimiques, comme agents capteurs de radicaux et comme agents antioxydants.

La préparation des diènes polysubstitués selon l'invention peut s'opérer de différentes façons. On peut, par exemple, utiliser comme intermédiaire un dérivé phosphoré. Cette préparation est particulièrement recommandée quand X représente un groupement $OR^3$. On fait subir à un composé de formule $X - CH_2 - Y$ une réaction de bromation, suivie d'une transposition appelée réaction d'Arbusov-Michaelis consistant à faire agir un trialkylphosphite :

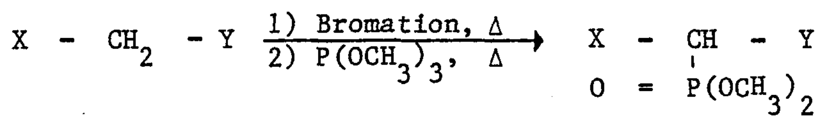


La réaction de bromation peut être effectuée à l'aide de brome ou de N-bromosuccinimide (NBS) pendant une durée de quelques heures à une température avoisinant 40°C. La réaction d'Arbusov-Michaelis s'opère en quelques heures à une température voisine de 80°C. Des exemples d'une telle préparation ont été décrits par Dinizo, Friersken, Pobst et Watt (Journal of Organic Chemistry, Volume 41, N°17, 1976).

Dans la deuxième étape, le composé phosphonate ainsi obtenu conduira aux diènes selon l'invention via une réaction de Wittig-Horner :

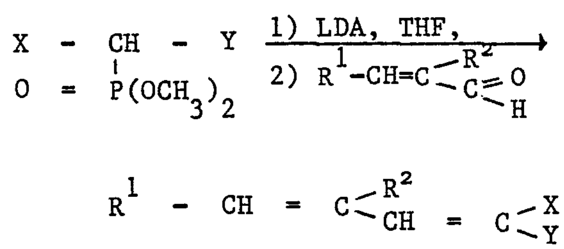


Une telle réaction est réalisée sous atmosphère d'argon à l'intervention de diisopropylamidure de lithium (LDA) dissous dans du tétrahydrofuranne (THF). On refroidit la solution de LDA et on ajoute lentement le phosphonate 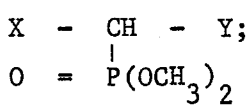

le mélange est alors agité pendant trente-cinq minutes. La solution est alors à nouveau refroidie à -78°C, température à laquelle on ajoute l'aldéhyde

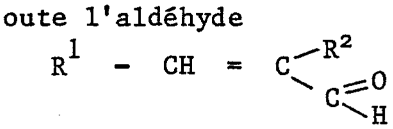

On laisse ensuite la température remonter progressivement. Après une nuit d'agitation, on additionne de l'eau au mélange, extrait plusieurs fois à l'éther et lave les phases éthérées à l'eau saturée en chlorure de sodium. Celles-ci sont séchées sur sulfate de magnésium puis concentrées avant distillation du produit final sous pression réduite.

Un autre mode de préparation est applicable quand X représente un groupement $SR^3$ et Y un groupement 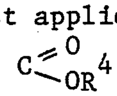.

Il consiste à faire réagir le composé de formule

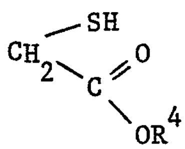

avec le 3 bromopropyne en présence d'éther et de triéthylamine selon l'équation

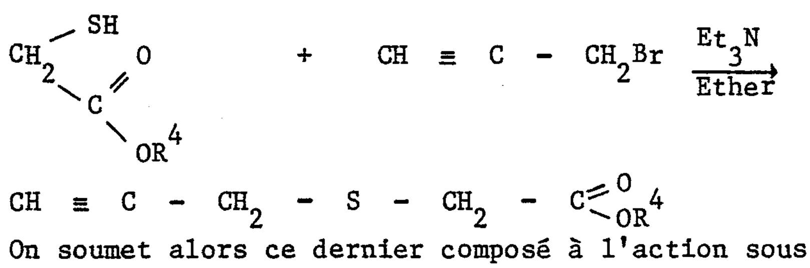


On soumet alors ce dernier composé à l'action sous argon du diméthylsulfate pendant 3 heures à 75°C puis à l'action d'un composé $NaOR^3$ dans de l'alcool $R^3OH$ à température normale pendant une nuit.

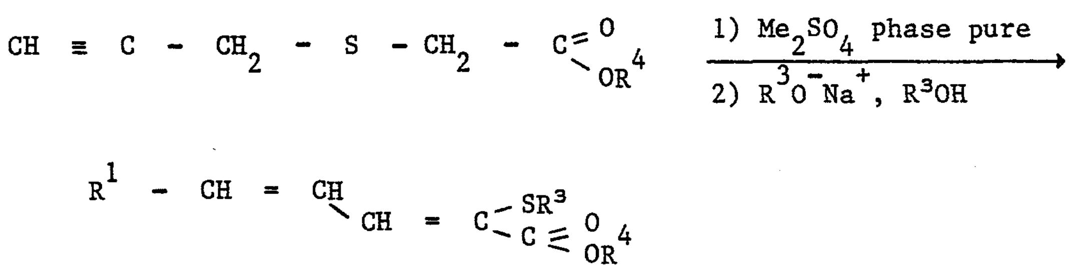


Après une nuit d'agitation à température ambiante, on additionne 30 ml d'eau à la solution, extrait plusieurs fois à l'éther et lave les phases éthérées à l'eau saturée en NaCl. On sèche sur $MgSO_4$ et concentre le filtrat.

Un troisième mode de préparation est applicable quand X représente un groupement $SR^3$ et Y représente un groupement $C \equiv N$.

On part d'un mélange composé de bromure d'allyle disubstitué de formule

$$\underset{R^1}{\underset{|}{CH}} = \underset{R^2}{\underset{|}{C}} - CH_2Br,$$

d'un dérivé méthylénique de formule $CH_2{<}\begin{smallmatrix} SR^3 \\ C \equiv N \end{smallmatrix}$ et de chlorure de triéthylbenzylammonium (TEBAC) auquel on additionne goutte à goutte une solution aqueuse de NaOH à 50%. Après agitation à température ambiante pendant une nuit, le mélange est ensuite dilué à l'eau et extrait à l'éther. Les phases organiques sont alors séchées sur du sulfate de magnésium $MgSO_4$. Après évaporation du solvant, une distillation fractionnée permet de recueillir le composé répondant à la formule

$$R^1 - CH = \overset{R^2}{\overset{|}{C}} - CH_2 - CH \begin{smallmatrix} \diagup SR^3 \\ \diagdown C \equiv N \end{smallmatrix}$$

et d'éliminer les produits de départ et de dialkylation. Ce dernier composé est ensuite dissous dans du dichlorométhane sec. On additionne goutte à goutte à la solution du chlorure de sulfuryle dissous dans le dichlorométhane. Le mélange ainsi obtenu est agité jusqu'à fin de dégagement gazeux. On évapore le solvant et distille le produit sous pression réduite. On a alors obtenu un dérivé chloré répondant à la formule

$$R^1 - CH = \overset{R^2}{\overset{|}{C}} - CH_2 - C \begin{smallmatrix} \diagup SR^3 \\ - Cl \\ \diagdown C \equiv N \end{smallmatrix}$$

On fait ensuite refluer ce dérivé chloré dans du dichlorométhane sec en présence de triéthylamine afin de procéder à sa déshydrochloration. Après évaporation du solvant, on traite le résidu avec de l'éther sec, filtre et évapore le solvant. Le produit selon l'invention est ensuite obtenu par distillation sous pression réduite.

Les exemples suivants illustrent l'invention.

Exemple 1 - Préparation du méthoxy-2-pentadiène-2,4-oate de méthyle

La réaction est effectuée sous atmosphère d'argon.

Dans un ballon à trois cols, séché à la flamme, muni d'une entrée d'argon, d'une ampoule à addition et d'une trappe à huile de silicone connectée à un tube de potasse, on place $1.1.10^{-2}$ mole de diisopropylamine sèche. A 0°C on ajoute $1.05.10^{-2}$ mole de BuLi dissous dans l'hexane et enlève après cinq minutes le bain réfrigérant.

Après vingt minutes d'agitation, l'hexane est chassé sous vide de trompe à eau. Le diisopropylamidure de lithium est ainsi obtenu sous forme d'une poudre blanche que l'on dissout immédiatement dans du THF sec. Cette solution est refroidie à -78°C; on y additionne lentement $10^{-2}$ mole de phosphonate

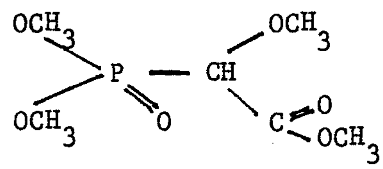

Le mélange est agité pendant 35 minutes. La solution est à nouveau refroidie à -78°C température à laquelle on ajoute $1,05.10^{-2}$ mole d'acroléine 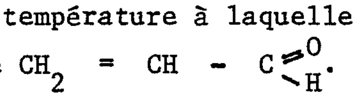. On laisse remonter progressivement la température. Après une nuit d'agitation, on additionne de l'eau au mélange, extrait plusieurs fois à l'éther et lave les phases éthérées à l'eau saturée en NaCl. Celles-ci sont séchées sur $MgSO_4$ puis concentrées. Le méthoxy-2-pentadiène-2,4-oate de méthyle est distillé à 60°C à une pression de 0,2 mm de Hg. On obtient un rendement de 64 %.

Le spectre infrarouge mesuré dans le dichlorométhane révèle des bandes d'absorption à 3 080 $cm^{-1}$, 2 840 $cm^{-1}$, 1 720 $cm^{-1}$, 1 625 $cm^{-1}$ et 1 580 $cm^{-1}$.

La spectrométrie de masse observe des ions de masse de 142, 127 et 83 daltons.

Les spectres de résonance magnétique nucléaire <u>RMN $^{13}$C</u> à 20 MHZ du méthoxy-2-pentadiène-2,4-oate de méthyle dissous dans le chloroforme deutéré confirment l'obtention de deux isomères dans une proportion pouvant être estimée à 60 % de E et 40 % de Z. On obtient en effet les caractéristiques suivantes.

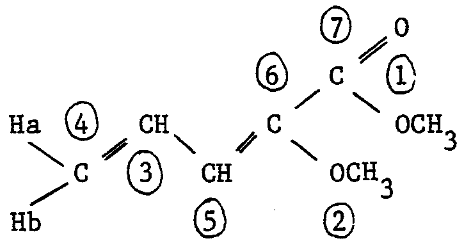

ISOMERE E

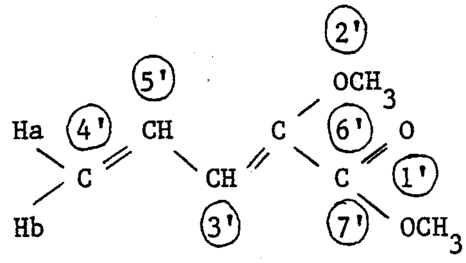

ISOMERE Z

Dans les divers exemples, les atomes de carbone sont numérotés par ordre de déplacement chimique en RMN. J est la constante de couplage dont la valeur est exprimée en Hertz. Par isomère E et isomère Z, on entend les isomères tels que définis dans l'article de J.E. Blackwood, C.L. Gladys, K.L. Loening, A.E. Petrarca et J.E. Rush (Journal of American Chemical Society, 1968, volume 90, page 509).

ISOMERE E

| | δ ppm | mult. | J |
|---|---|---|---|
| 1 | 51,5 | Q, s | $^1J$ = 147,5 |
| 2 | 55,2 | Q, s | $^1J$ = 144,7 |
| 3 | 113,7 | D, m | $^1J$ = 154 |
| 4 | 118,8 | DD,d | $^1J$4-a(b)=154,4<br>$^1J$4-b(a)=160,2<br>$^3J$ = 5,9 |
| 5 | 131,4 | D, d | $^1J$ = 159,3 |
| 6 | 145,5 | S, m | |
| 7 | 163,0 | S, m | |

ISOMERE Z

| | δ ppm | mult. | J |
|---|---|---|---|
| 1' | 51,5 | Q, s | $^1J$ = 147,5 |
| 2' | 59,9 | Q, s | $^1J$ = 144,7 |
| 3' | 125,2 | S, m | $^1J$ = 161 |
| 4' | 122,1 | DD,dd | $^1J$4'-a(b)=153,2<br>$^2J$ = 2,5<br>$^1J$4'-b(a)=164<br>$^2J$ = 6,7 |
| 5' | 129,4 | D, s | $^1J$ = 155,3 |
| 6' | 145,1 | S, m | |
| 7' | 163,8 | S, m | |

mult. désigne l'abréviation de multiplicité

Le spectre U.V. dans l'hexane révèle une longueur d'onde maximale de 263 nm; le coefficient d'extinction ε est égal à 15 500.

Enfin l'analyse élémentaire donne les résultats suivants :

| | | | |
|---|---|---|---|
| théorique : | C : 59,14 | H : 7,09 | O : 33,76 |
| expérimental : | C : 59,21 | H : 7,00 | O : 33,79 |

Exemple 2 - Préparation du diméthoxy-2,4-pentadiène-2,4-oate de méthyle

La réaction est effectuée sous atmosphère d'argon.

Dans un ballon à trois cols, séché à la flamme, muni d'une entrée d'argon, d'une ampoule à addition et d'une trappe à huile de silicone connectée à un tube de potasse, on place $1,1.10^{-2}$ mole de diisopropylamine sèche. A 0°C on ajoute $1,05.10^{-2}$ mole de BuLi dissous dans l'hexane et enlève après cinq minutes le bain réfrigérant.

Après vingt minutes d'agitation, l'hexane est chassé sous vide de trompe à eau. Le diisopropylamidure de lithium est ainsi obtenu sous forme d'une poudre blanche que l'on dissout immédiatement dans du THF sec. Cette solution est refroidie à -78°C; on y additionne lentement $10^{-2}$ mole de phosphonate

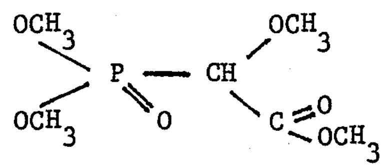

Le mélange est agité pendant 35 minutes. La solution est à nouveau refroidie à -78°C température à laquelle on ajoute $1,05.10^{-2}$ mole de 2-méthoxy-acroléine

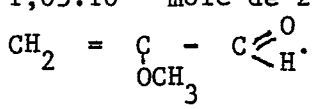

On laisse remonter progressivement la température. Après une nuit d'agitation, on additionne de l'eau au mélange, extrait plusieurs fois à l'éther et lave les phases éthérées à l'eau saturée en NaCl. Celles-ci sont séchées sur $MgSO_4$ puis concentrées. Le diméthoxy-2,4-pentadiène-2,4-oate de méthyle est distillé à 105°C à une pression de 12 mm de Hg. On obtient un rendement supérieur à 90 %.

Le spectre infrarouge mesuré dans le dichlorométhane révèle des bandes d'absorption à 3 050 $cm^{-1}$, 2 850 $cm^{-1}$, 1 745 $cm^{-1}$, 1 645 $cm^{-1}$ et 1 590 $cm^{-1}$.

La spectrométrie de masse observe des ions de masse de 172 daltons.

Le spectre de résonance magnétique nucléaire $RMN^{13}C$ à 20 MHZ du diméthoxy-2,4-pentadiène-2,4-oate de méthyle dissous dans le chloroforme deutéré donne les résultats suivants :

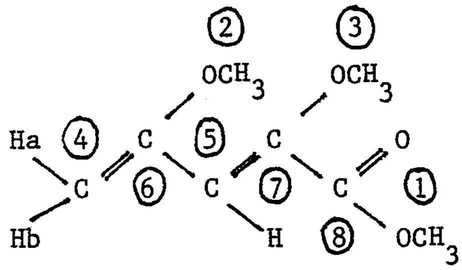

| | δ ppm | mult. | J |
|---|---|---|---|
| 1 | 51,9 | Q, s | $^1J$ = 147,4 |
| 2 | 54,4 | Q, s | $^1J$ = 143,7 |
| 3 | 59,5 | Q, s | $^1J$ = 145,4 |
| 4 | 89,2 | DD,d | $^1J$4-a(b) = 160 |
| | | | $^1J$4-b(a) = 164,8 |
| 5 | 119,8 | D,dd | $^1J$ = 160 |
| 6 | 145,7 | S, m | |
| 7 | 156,0 | S, m | |
| 8 | 164,3 | S (quint.) | |

La spectrométrie U.V. dans l'hexane révèle une longueur d'onde maximale de 279 nm; ε = 8 900.

Exemple 3 - Préparation du méthoxy-2-pentadiène-2,4-nitrile

La réaction est effectuée sous atmosphère d'argon.

Dans un ballon à trois cols, séché à la flamme, muni d'une entrée d'argon, d'une ampoule à addition et d'une trappe à huile de silicone connectée à un tube de potasse, on place $1,1.10^{-2}$ mole de diisopropylamine sèche. A 0°C on ajoute $1,05.10^{-2}$ mole de BuLi dissous dans l'hexane et enlève après cinq minutes le bain réfrigérant.

Après vingt minutes d'agitation, l'hexane est chassé sous vide de trompe à eau. Le diisopropylamidure de lithium est ainsi obtenu sous forme d'une poudre blanche que l'on dissout immédiatement dans du THF sec. Cette solution est refroidie à -78°C; on y additionne lentement $10^{-2}$ mole de phosphonate

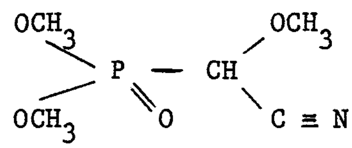

Le mélange est agité pendant 35 minutes. La solution est à nouveau refroidie à -78°C température à laquelle on ajoute $1,05.10^{-2}$ mole d'acroléine $CH_2 = CH - C\begin{smallmatrix}\nearrow O\\ \searrow H\end{smallmatrix}$. On laisse remonter progressivement la température. Après une nuit d'agitation, on additionne de l'eau au mélange, extrait plusieurs fois à l'éther et lave les phases éthérées à l'eau saturée en NaCl. Celles-ci sont séchées sur $MgSO_4$ puis concentrées et distillées à 70°C à une pression de 12 mm de Hg. On obtient un rendement de 70 % en méthoxy-2-pentadiène-2,4-nitrile qui se présente comme un liquide incolore.

Le spectre infrarouge mesuré dans le dichlorométhane révèle des bandes d'absorption à 3 100 $cm^{-1}$, 2 850 $cm^{-1}$, 2 235 $cm^{-1}$ et 2 215 $cm^{-1}$ (C ≡ N), 1 625 $cm^{-1}$ et 1 595 $cm^{-1}$ (C = C).

La spectrométrie de masse observe des ions de masse de 109 et 94 daltons.

Les spectres de résonance magnétique nucléaire <u>RMN $^{13}$C</u> à 50 MHZ du méthoxy-2-pentadiène-2,4-nitrile dissous dans le chloroforme deutéré confirment l'obtention de deux isomères (dans une proportion pouvant être estimée à 75 % de E et 25 % de Z); on trouve en effet les caractéristiques suivantes :

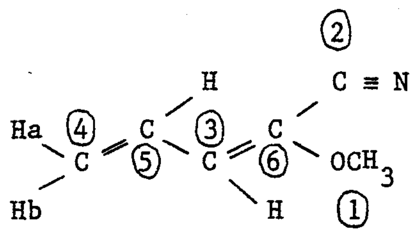

ISOMERE E

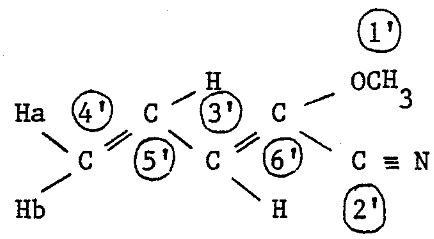

ISOMERE Z

| ISOMERE E | δ ppm | mult. | J | ISOMERE Z | δ ppm | mult. | J |
|---|---|---|---|---|---|---|---|
| 1 | 57,0 | Q, s | $^1J$ = 145,8 | 1' | 58,7 | Q, s | $^1J$ = 145,8 |
| 2 | 113,1 | S, d | $^3J$ = 11,2 | 2' | 114,2 | S, d | $^3J$ = 4,7 |
| 3 | 119,7 | D, m | | 3' | 124,2 | D, m | $^1J$ = 161<br>$^2J$ = 3,4<br>$^3J$3'-4'a(b)=14,5<br>$^3J$3'-4'b(a)=9 |
| 4 | 120,8 | DD,d | $^1J$4-a(b)=156,5<br>$^1J$4-b(a)=161,5<br>$^3J$4 = 5,1 | 4' | 121,7 | DD,m | $^1J$4'-a(b)=156,9<br>$^1J$4'-b(a)=161,7<br>$^3J$ = 4,9 |
| 5 | 130,0 | D, s.el | $^1J$ = 153,4 | 5' | 128,0 | D,el. | $^1J$ = 159<br>$^2J$ = 3 |
| 6 | 132,5 | S, q | $^3J$ = 4,5 | 6' | 128,8 | S, m | |

Le spectre U.V. dans l'hexane révèle une longueur d'onde maximale de 257 nm; ε = 16 500.

Enfin l'analyse élémentaire donne les résultats suivants :

théorique : C : 66,04 H : 6,46 N : 12,83

expérimental : C : 66,08 H : 6,39 N : 12,93

Exemple 4 - Préparation du méthylthio-2-pentadiène -2,4-nitrile

A un mélange composé de 6,05 g ($5.10^{-2}$ mole) de bromure d'allyle, 4,35 g ($5.10^{-2}$ mole) de méthylthio-acétonitrile et 0,3 g de TEBAC, on ajoute goutte à goutte 20 ml d'une solution aqueuse de NaOH à 50 %. Après agitation à température ambiante pendant une nuit, le mélange est dilué à l'eau et extrait à l'éther.

Les phases organiques sont séchées sur $MgSO_4$. Après évaporation du solvant, une distillation fractionnée à 104°C sous une pression de 40 mm de mercure permet d'obtenir le méthylthio-2-pentène-4-nitrile avec un rendement variant entre 30 % et 60 %. Le spectre infrarouge du méthylthio-2-pentène-4-nitrile révèle des bandes d'absorption à 3 100 $cm^{-1}$, 2 850 $cm^{-1}$, 2 250 $cm^{-1}$ et 1 650 $cm^{-1}$; la spectrométrie de masse observe un ion de masse à 127 daltons.

$2.10^{-2}$ moles de méthylthio-2-pentène-4-nitrile sont ensuite dissoutes dans 50 ml de $CH_2Cl_2$ sec. On y additionne goutte à goutte $2.10^{-2}$ moles de $SO_2Cl_2$ en solution dans 10 ml de $CH_2Cl_2$. Le mélange est agité jusqu'à fin de dégagement gazeux (trappe à huile de silicone). On évapore le solvant et on distille à 82°C sous une pression de 12 mm de mercure. On obtient du chloro-2-méthylthio-2-pentène-4-nitrile avec un rendement supérieur à 95 %.

Enfin 1,61 g ($10^{-2}$ mole) de chloro-2-méthylthio-2-pentène-4-nitrile sont reflués pendant 20 heures dans 30 ml de $CH_2Cl_2$ sec, en présence de 2,02 g (2 eq) de triéthylamine. Après évaporation du solvant, on traite le résidu avec 4 fractions de 30 ml d'éther sec, filtre et évapore le solvant. Le produit est distillé à 40°C sous une pression de 0,05 mm de Hg.

On obtient le méthylthio-2-pentadiène-2,4-nitrile avec un rendement avoisinant 90 %.

Le spectre infrarouge mesuré dans le dichlorométhane révèle des bandes d'absorption à 3 100 $cm^{-1}$, 2 850 $cm^{-1}$, 2 230 $cm^{-1}$, 1 615 $cm^{-1}$ et 1 575 $cm^{-1}$.

La spectrométrie de masse observe des ions de masse de 125 et 110 daltons.

Les spectres de résonance magnétique nucléaire <u>RMN $^{13}C$</u> à 20 MHZ du méthylthio-2-pentadiène-2,4-nitrile dissous dans le chloroforme deutéré confirment l'obtention de deux isomères dans une proportion pouvant être estimée à 65 % de E et 35 % de Z.

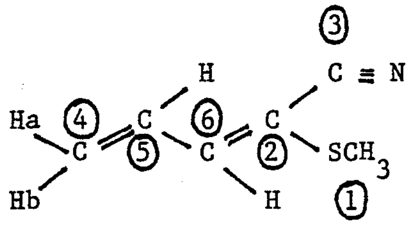

ISOMERE E

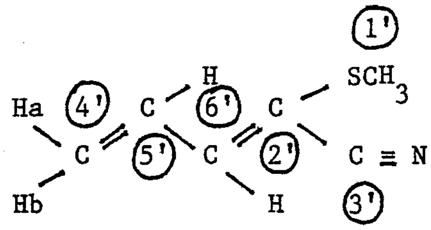

ISOMERE Z

| ISOMERE E | | | | ISOMERE Z | | | |
|---|---|---|---|---|---|---|---|
| | δ ppm | mult. | J | | δ ppm | mult. | J |
| 1 | 15,9 | Q, s | $^1J = 140,8$ | 1' | 16,0 | Q, s | $^1J = 141,6$ |
| 2 | 110,2 | S, m | | 2' | 110,2 | S, m | |
| 3 | 113,7 | S (d) | $^3J = 10,3$ | 3' | 115,5 | S | $^3J = 4$ |
| 4 | 123,9 | DD,m | $^1J = 157,9$ | 4' | 125,6 | DD,m | $^1J = 157,5$ |
| 5 | 132,2 | D, s | $^1J = 155,5$ | 5' | 130,7 | D, s | $^1J = 156,5$ |
| 6 | 142,8 | D, m | $^1J = 162$ | 6' | 143,2 | D, m | $^1J = 161$ |

Exemple 5 - Préparation du méthoxy-2-méthyl-4-pentadiène -2,4-oate de méthyle

La réaction est effectuée sous atmosphère d'argon.

Dans un ballon à trois cols, séché à la flamme, muni d'une entrée d'argon, d'une ampoule à addition et d'une trappe à huile de silicone connectée à un tube de potasse, on place $1,1.10^{-2}$ mole de diisopropylamine sèche. A 0°C on ajoute $1,05.10^{-2}$ mole de BuLi dissous dans l'hexane et enlève après cinq minutes le bain réfrigérant.

Après vingt minutes d'agitation, l'hexane est chassé sous vide de trompe à eau. Le diisopropylamidure de lithium est ainsi obtenu sous forme d'une poudre blanche que l'on dissout immédiatement dans du THF sec. Cette solution est refroidie à -78°C; on y additionne lentement $10^{-2}$ mole de phosphonate

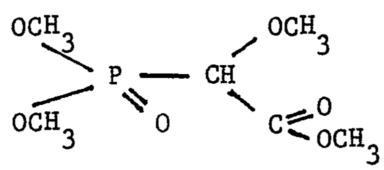

Le mélange est agité pendant 35 minutes. La solution est à nouveau refroidie à -78°C température à laquelle on ajoute $1,05.10^{-2}$ mole de 2-méthylacroléine

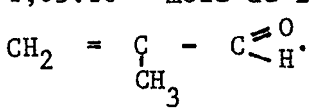

On laisse remonter progressivement la température. Après une nuit d'agitation, on additionne de l'eau au mélange, extrait plusieurs fois à l'éther et lave les phases éthérées à l'eau saturée en NaCl.

Celles-ci sont séchées sur $MgSO_4$ puis concentrées. Le méthoxy-2-méthyl-4-pentadiène-2,4-oate de méthyle est distillé à 45°C à une pression de 0,05 mm de Hg. On obtient un rendement de 80 %.

Le spectre infrarouge mesuré dans le dichlorométhane révèle des bandes d'absorption à 3 080 $cm^{-1}$, 2 830 $cm^{-1}$, 1 720 $cm^{-1}$, 1 630 $cm^{-1}$ et 1 595 $cm^{-1}$.

La spectrométrie de masse observe des ions de masse de 156, 141 et 97 daltons.

Les spectres de résonance magnétique nucléaire RMN $^{13}C$ à 20 MHZ du méthoxy-2-méthyl-4-pentadiène-2,4-oate de méthyle dissous dans le chloroforme deutéré confirment l'obtention de deux isomères dans une proportion pouvant être estimée à 75 % de E et 25 % de Z. On obtient en effet les caractéristiques suivantes.

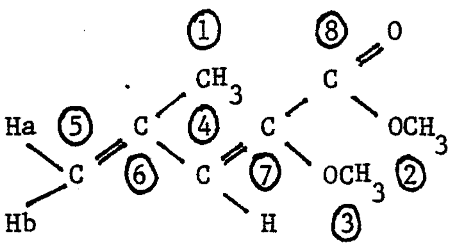

ISOMERE E

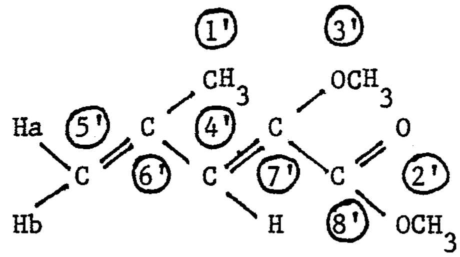

ISOMERE Z

| | δ ppm | mult. | J |
|---|---|---|---|
| 1 | 20,9 | Q, m | |
| 2 | 50,9 | Q, s | $^{1}J$ = 147,5 |
| 3 | 54,6 | Q, s | $^{1}J$ = 144,5 |
| 4 | 108,2 | D, m | $^{1}J$ = 157 |
| 5 | 114,1 | DD, quint | $^{1}J5-a(b)$=156,6 $^{3}J$ = 5,6 |
| 6 | 138,1 | S, m | |
| 7 | 147,2 | S, m | |
| 8 | 164,0 | S,dq | $^{3}J$ = 11 |

| | δ ppm | mult. | J |
|---|---|---|---|
| 1' | 20,5 | Q, m | |
| 2' | 50,9 | Q, s | $^{1}J$ = 146 |
| 3' | 58,9 | Q, s | $^{1}J$ = 145,2 |
| 4' | 125,1 | D, m | $^{1}J$ = 158 |
| 5' | 114,1 | DD, quint | $^{1}J5'-a(b)$=156,6 $^{3}J$ = 5,6 |
| 6' | 138,8 | S, m | |
| 7' | 144,1 | S, m | |
| 8' | 163,8 | S, m | |

Le spectre U.V. dans l'hexane révèle une longueur d'onde maximale de 273 nm (ε = 27 700).

Exemple 6 - Préparation du méthoxy-2-méthyl-4-pentadiène-2,4-nitrile

La réaction est effectuée sous atmosphère d'argon.

Dans un ballon à trois cols, séché à la flamme, muni d'une entrée d'argon, d'une ampoule à addition et d'une trappe à huile de silicone connectée à un tube de potasse, on place $1,1.10^{-2}$ mole de diisopropylamine sèche. A 0°C on ajoute $1,05.10^{-2}$ mole de BuLi et enlève après cinq minutes le bain réfrigérant.

Après vingt minutes d'agitation, l'hexane est chassé sous vide de trompe à eau. Le diisopropylamidure de lithium est ainsi obtenu sous forme d'une poudre blanche que l'on dissout immédiatement dans du THF sec. Cette solution est refroidie à -78°C; on y additionne lentement $10^{-2}$ mole de phosphonate

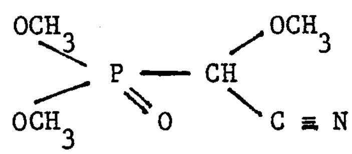

Le mélange est agité pendant 35 minutes. La solution est à nouveau refroidie à -78°C température à laquelle on ajoute $1,05.10^{-2}$ mole de 2-méthyl-acroléine

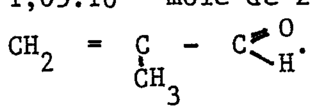

On laisse remonter progressivement la température. Après une nuit d'agitation, on additionne de l'eau au mélange, extrait plusieurs fois à l'éther et lave les phases éthérées à l'eau saturée en NaCl. Celles-ci sont séchées sur $MgSO_4$ puis concentrées. Le méthoxy-2-méthyl-4-pentadiène-2,4-oate de méthyle est distillé à 83°C à une pression de 12 mm de Hg. On obtient un rendement de 86 % en méthoxy-2-méthyl-4-pentadiène-2,4-nitrile qui se présente comme un liquide incolore.

Le spectre infrarouge mesuré dans le dichlorométhane révèle des bandes d'absorption à 3 100 $cm^{-1}$, 2 850 $cm^{-1}$, 2 230 $cm^{-1}$ et 2 220 $cm^{-1}$, 1 620 $cm^{-1}$ et 1 598 $cm^{-1}$.

Par la spectrométrie de masse on observe un ion de masse de 123 daltons.

Les spectres de résonance magnétique nucléaire RMN $^{13}C$ à 20 MHZ du méthoxy-2-méthyl-4-pentadiène-2,4-nitrile dissous dans le chloroforme deutéré confirment l'obtention de deux isomères dans une

proportion pouvant être estimée à 65 % de E et 35 % de Z. On trouve en effet les caractéristiques suivantes.

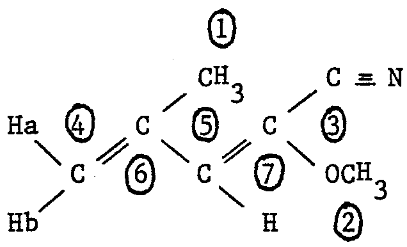

ISOMERE E

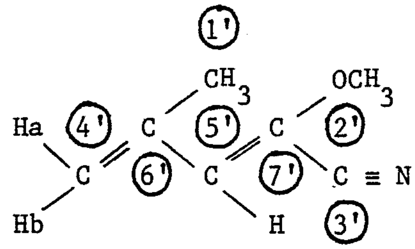

ISOMERE Z

| | δ ppm | mult. | J | | δ ppm | mult. | J |
|---|---|---|---|---|---|---|---|
| 1 | 20,5 | Q, ddd | $^1J$ = 127,3<br>$^3J$1-4b=10,7<br>$^3J$1-4a(b)=4,3<br>$^3J$1-5(4a)=6,2 | 1' | 21,8 | Q, m | |
| 2 | 57,0 | Q, s | ($^1J$)= 145,5 | 2' | 58,6 | Q, s | $^1J$ = 145,7 |
| 3 | 114,2 | S, d | $^3J$ = 11,9 | 3' | 114,2 | S, d | |
| 4 | 120,4 | DD, quint | $^1J$ = 158,05<br>$^3J$ = 6 | 4' | 121,2 | DD, m | |
| 5 | 122,8 | D, m | $^1J$ = 154,5 | 5' | 125,1 | D, m | |
| 6 | 129,2 | S, m | | 6' | 128,1 | S, m | |
| 7 | 136,6 | S, q.él. | $^3J$ = 7 | 7' | 138,5 | S, m | |

Le spectre U.V. dans l'hexane révèle une longueur d'onde maximale de 260 nm (ε = 14 300).

# REVENDICATIONS

1 - Diènes polysubstitués caractérisés en ce qu'ils répondent à la formule générale :

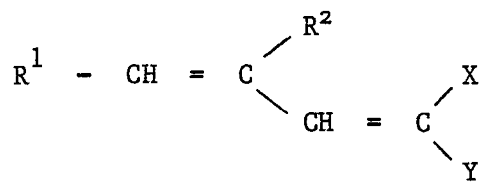

dans laquelle :

- X représente un groupement sélectionné parmi les groupements SH, $SR^3$, $OR^3$, $SeR^3$, $NHR^3$ et $N = R^3$ dans lesquels $R^3$ représente un groupement aliphatique ou cyclique, saturé ou non, comptant de 1 à 20 atomes de carbone,
- Y représente un groupement sélectionné parmi les groupements $C \equiv N$ et $C\begin{smallmatrix}{\nearrow}O\\ {\searrow}OR^4\end{smallmatrix}$ où $R^4$ représente l'hydrogène ou un groupement aliphatique ou cyclique, saturé ou non, comptant de 1 à 20 atomes de carbone, sous réserve que lorsque X représente le groupement $SCH_3$, Y ne représente pas le groupement $C\begin{smallmatrix}{\nearrow}O\\ {\searrow}OC_2H_5\end{smallmatrix}$ et que lorsque X représente un groupement $OR^3$ où R3 est un groupement alkyle comprenant au moins trois atomes de carbone, Y ne représente pas le groupement $C \equiv N$,
- $R^1$ représente l'hydrogène, un halogène ou un groupement aliphatique, substitué ou non, comptant de 2 à 8 atomes de carbone,
- $R^2$ représente l'hydrogène, un halogène, un groupement aliphatique, substitué ou non, et comptant de 1 à 8 atomes de carbone ou un groupement aromatique, substitué ou non, et comptant de 5 à 8 atomes de carbone.

2 - Diènes polysubstitués selon la revendication 1, caractérisés en ce que X représente un groupement sélectionné parmi les groupements $SR^3$, $OR^3$ et $NHR^3$, $R^4$ représente l'hydrogène ou un groupement alkyle, cycloalkyle, alcényle ou cycloalcényle, substitué ou non et comptant jusqu'à 12 atomes de carbone ou un groupement phényle substitué ou non comptant de 6 à 10 atomes de carbone, $R^1$ représente l'hydrogène, ou un groupement alkyle, alcoxy, alcényle ou alcényloxy, substitué ou non, et comptant de 2 à 6 atomes de carbone, $R^2$ repré-

sente l'hydrogène ou un groupement alkyle, alcoxy, alcényle ou alcényloxy, substitué ou non, et comptant de 1 à 6 atomes de carbone ou un groupement phényle, phénoxy, phénylalkyle ou phénylalcoxy substitué ou non et comptant de 5 à 8 atomes de carbone.

3 - Diènes polysubstitués selon l'une quelconque des revendications 1 ou 2, caractérisés en ce que $R^3$ représente un groupement alkyle, substitué ou non, comptant de 1 à 6 atomes de carbone.

4 - Diènes polysubstitués selon l'une quelconque des revendications 1 à 3, caractérisés en ce que Y représente un groupement sélectionné parmi les groupements $C \equiv N$ et $C\begin{smallmatrix}\nearrow O \\ \searrow OR^4\end{smallmatrix}$ où $R^4$ représente l'hydrogène ou un groupement alkyle, substitué ou non, comptant de 1 à 3 atomes de carbone ou un groupement phényle substitué ou non comptant de 6 à 8 atomes de carbone.

5 - Diènes polysubstitués selon l'une quelconque des revendications 1 à 4 caractérisés en ce que $R^1$ représente l'hydrogène ou un groupement alkyle ou alcényle comptant de 2 à 4 atomes de carbone et $R^2$ représente l'hydrogène, un groupement méthoxy, un groupement alkyle comptant de 1 à 3 atomes de carbone ou un groupement phényle comptant six atomes de carbone.

6 - Diènes polysubstitués selon l'une quelconque des revendications 1 à 5, caractérisés en ce que X représente un groupement sélectionné parmi les groupements $OCH_3$ et $SCH_3$.

7 - Diènes polysubstitués selon l'une quelconque des revendications 1 à 6, caractérisés en ce que Y représente un groupement sélectionné parmi les groupements $C \equiv N$ et $C\begin{smallmatrix}\nearrow O \\ \searrow OCH_3\end{smallmatrix}$.

8 - Diènes polysubstitués selon l'une quelconque des revendications 1 à 7, caractérisés en ce que $R^1$ et $R^2$ représentent l'hydrogène.

9 - Diènes polysubstitués selon l'une quelconque des revendications 1 à 8, caractérisés en ce que X représente le groupement $OCH_3$.

10 - Diènes polysubstitués selon l'une quelconque des revendications 1 à 9, caractérisés en ce que Y représente le groupement